# EUROPEAN PATENT APPLICATION

(11) **EP 3 714 704 A1**
(43) Date of publication of application: **30.09.2020**
(21) Application number: 20163017.5
(22) Date of filing: 13.03.2020
(51) Int. Cl.: A24B 15/12, A24F 40/00

(54) **CONCENTRATE DISPENSER**

(30) Priority: 14.03.2019 US 201962818257 P; 22.05.2019 US 201962851152 P
(71) Applicant: Canopy Growth Corporation, Smiths Falls, Ontario K7A 0A8 (CA)
(72) Inventor: JONES, Mark, TORONTO, ONTARIO M6K 3R4 (CA); STEWART, Andrew, OTTAWA, ONTARIO K2J 1G8 (CA); TOLLS, Colin, OTTAWA, ONTARIO K2A 1G5 (CA); VERMETTE, Yan, OTTAWA, ONTARIO K2K 0A9 (CA)
(74) Representative: Murgitroyd & Company

(57) **Abstract**

A dispenser with a housing, a plunger, and an activation mechanism coupled to the plunger. The housing defines a reservoir configured to retain a liquid and a dispensing orifice in fluid communication with the reservoir. The plunger is configured to move through the reservoir toward the dispensing orifice to dispense the liquid through the dispensing orifice. The activation mechanism is configured to be moved by a user, and movement of the activation mechanism in a first direction causes the plunger to move toward the dispensing orifice. At least one of the activation mechanism and the housing is configured to provide feedback to a user when the activation mechanism moves a predetermined distance in the first direction. A method of using the dispenser.

## Description

### CROSS-REFERENCE TO RELATED APPLICATIONS

This application is based on and claims priority to U.S. Provisional Application Serial No. 62/818,257 filed on March 14, 2019 and U.S. Provisional Application Serial No. 62/851,152 filed on May 22, 2019, each of which is incorporated herein by reference in its entirety.

### BACKGROUND OF THE INVENTION

### 1. FIELD OF THE INVENTION

The present invention relates, generally, to dispensing devices, and more specifically to a device for dispensing a viscous fluid or concentrate.

### 2. DESCRIPTION OF RELATED ART

In recent years, studies have begun to provide evidence that cannabis may be useful in treating many age-related diseases such as cognitive decline, arthritis and insomnia. It is important to maintain such cannabis safe from ingestion by children.

To utilize cannabis concentrate extract, the concentrate is traditionally transferred from its storage container to a system that vaporizes the cannabis concentrate. This process, known to some as dabbing, typically uses a dentistry like tool for scraping the wax like substance to an extremely hot object (sometimes referred to as a rig), then inhaling the vapors that are produced. In use, a rig may reach temperatures of anywhere up to 537° C (1000° F). Others may use a syringe like tool to transfer the concentrate to a preheated object and inhale the vapors that are produced. Transferring the cannabis concentrate to another system, however, can be difficult. For example, the concentrate typically has sticky or tacky physical characteristics that make transfer cumbersome. Therefore, a tool is needed to transfer the concentrate to another system for use.

Existing devices such as syringe-like instruments are not suited for dispensing a precise and repeatable amount of the concentrate. These devices also carry negative connotations of narcotics like heroin. Existing bottom twist plunger devices again, do not allow precise dispensing and are difficult to use with one hand, as it is difficult to apply enough torque using the round housing and twisting knob to dispense a viscous liquid.

Patent application US2018/0327173 describes a device that aims to solve such problems. However, the mechanism is relatively complicated and requires an intricate assembly of many parts that need to be manufactured to precise tolerances. Additionally, the device requires two steps to dispense the concentrate, the first of which appears to require two hands. If the initial amount of the concentrate is underestimated, the two-step procedure would need to be executed again.

Patent application US2018/0361066 describes a device that uses a stepping or servo electric motor to drive a plunger. This type of device is much more expensive to manufacture and requires batteries or another source of electricity.

In many applications, reclosable containers are designed with child safety as a paramount consideration, given various incidents of adverse reaction to the consumption of marijuana involving children. An example is child-safe cannabis containers.

The most common solution for designing child-safe or child-resistant containers is a closure which has to be pushed down and turned simultaneously. The rationale is that young children have neither the strength nor the dexterity required for this operation. Safe use of such a packaging solution requires that the safety feature is restored to the same condition after reclosing the package. Practice shows however, that child-safe caps also present a challenge to elderly persons and people with reduced hand function. For them, child-resistant caps are hard to open, which causes the containers to be poorly reclosed, or even left open, which may be counterproductive and result in access to the contents of such containers by children.

The design of child resistant closures that are easy for elderly patients to operate is difficult because of the multitude of factors affecting the discrimination between children and adult physical and cognitive abilities. There are various factors to be considered in designing an effective child resistant, elderly friendly closure, and most of these factors may interact in nonlinear, unpredictable ways. These factors can manifest from the differences in palm size, finger length, skin friction, time to frustration, perception of operable elements, and medical conditions.

For a "push and turn" mechanism, the factors that must be considered, for example, include the texture, shape, diameter and height of the gripping surfaces, as each can differentially affect the abilities of different users in operating the mechanism. In terms of interaction with the physical characteristics of the user, they affect the ability of the user to apply a sufficient axial (pushing) force, as well as the simultaneously required rotational (twisting) force. Additionally, the friction at the interface between the different components inherent in the mechanism can be described as static or dynamic in nature, and depends on the different materials used in the container collar, the cap and the internal bias member. This will affect the force required to initiate, and continue the downward movement of the cap, as well as initiating and continuing the rotation of the cap. Furthermore, the dampening of the pushing motion can obfuscate the position at which the cap will be released to turn. Different users may use different parts of their hands to operate the mechanism, so the gripping force of the hand has to be considered. Where the mechanism is designed for one-handed operation, the forces that the user can apply with only the thumb and forefinger must be considered. The multitude of other factors include, but are not limited to, fine motor skills, hand steadiness, eye-hand coordination, vision, cognitive ability of the user, and the cues presented by the mechanism as to how it may operate.

A typical mechanism that embodies the use of simultaneous axial and twisting forces on a cap is exemplified in U.S. Patent No. 4,059,198.

Such a mechanism comprises the container body itself that has a plurality of hook-like protuberances arranged around the outside circumference of its open end, a cap that has a set of complementary nubs arranged around its inner circumference, such that the nubs can engage the hooks upon insertion of the container into the cap such that the nubs initially clear the hooks, but engage the hooks when the cap is rotated relative to the container body in a bayonet fashion. The key to the operation of the mechanism is a bias member conventionally a dome like circular member inserted into the cap that applies an upward counterforce to attempts to press the cap onto the open end of the container.

In the current art, the child resistant bayonet capped container bodies are usually cylindrical in shape, though rectangular prism shapes are known as well. Whether the container is cylindrical or prism shaped, the caps are round.

### BRIEF SUMMARY OF THE INVENTION

One aspect of the invention described herein is directed toward a dispenser with a housing, a plunger, and an activation mechanism coupled to the plunger. The housing defines a reservoir configured to retain a liquid and a dispensing orifice in fluid communication with the reservoir. The plunger is configured to move through the reservoir toward the dispensing orifice to dispense the liquid through the dispensing orifice. The activation mechanism is configured to be moved by a user, and movement of the activation mechanism in a first direction causes the plunger to move toward the dispensing orifice. At least one of the activation mechanism and the housing is configured to provide feedback to a user when the activation mechanism moves a predetermined distance in the first direction. The feedback allows a user to know when a certain amount of liquid is dispensed through the dispensing orifice as a result of movement of the activation mechanism.

The plunger may include a shaft with threads that engage a threaded surface of the activation mechanism, and wherein rotation of the activation mechanism in the first direction causes the plunger to move toward the dispensing orifice. The activation mechanism may have a knob and a sleeve, wherein the threaded surface of the activation mechanism is an interior surface of the sleeve. The shaft of the plunger may have a first section and a second section, the first section including the threads that engage the threaded surface of the activation mechanism. A spring may be positioned between the first section and the second section, the spring being operable to compress as the plunger moves toward the dispensing orifice. The plunger may include a shaft and a plunger tip coupled to the shaft, wherein the plunger tip is positioned in the reservoir, wherein the plunger tip is resilient, and wherein the plunger tip is configured to compress as the plunger moves toward the dispensing orifice.

One of the activation mechanism and the housing may include a tooth that engages a surface of the other of the activation mechanism and the housing to provide the feedback to the user. The feedback may be a tactile feedback or an audible feedback.

A cap may be releasably coupled to the housing for selectively covering and uncovering the dispensing orifice. A resilient seal may be coupled to the cap and configured to seal between the housing and the cap to inhibit fluid communication between the dispensing orifice and an ambient environment when the cap is coupled to the housing.

Another aspect of the invention described herein is directed toward a method for dispensing a liquid from the dispenser described above. The method includes moving the activation mechanism in a first direction to cause the plunger to move through the reservoir toward the dispensing orifice and dispense a portion of the liquid through the dispensing orifice; and receiving feedback from at least one of the activation mechanism and the housing when the activation mechanism has moved a predetermined distance in the first direction.

Additional aspects of the invention, together with the advantages and novel features appurtenant thereto, will be set forth in part in the description which follows, and in part will become apparent to those skilled in the art upon examination of the following, or may be learned from the practice of the invention. The objects and advantages of the invention may be realized and attained by means of the instrumentalities and combinations particularly pointed out in the appended claims.

### BRIEF DESCRIPTION OF THE DRAWINGS

The subject matter which is regarded as the invention is particularly pointed out and distinctly claimed in the claims at the conclusion of the specification. The foregoing and other features, and advantages of the invention will be readily understood from the following detailed description of preferred embodiments taken in conjunction with the accompanying drawings in which:
FIG. 1 is a perspective view of a dispenser in accordance with one embodiment of the invention disclosed herein;
FIG. 2 is a side cross-sectional view of the dispenser of FIG. 1 taken through the line 2-2 in FIG. 1;
FIG. 3 is a perspective view of the dispenser of FIG. 1 with a cap removed;
FIG. 4 is a perspective view of an activation mechanism, a reservoir, and a tip of the dispenser of FIG. 1;
FIG. 4A is a detail view showing the connection between the activation mechanism and the reservoir shown in FIG. 4;
FIG. 5 is a perspective view of the dispenser of FIG. 1 showing an opposite side of the dispenser as shown in FIG. 1;
FIG. 6 is a perspective view of an activation mechanism and a tip of a plunger of the dispenser of FIG. 1;
FIG. 7 is a perspective view of the plunger of the dispenser of FIG. 1;
FIG. 8 is a cross-sectional view taken through the line 8-8 of FIG. 5;
FIG. 8A is a cross-sectional view taken through the line 8A-8A of FIG. 5;
FIG. 9 is a perspective view of a top portion of the dispenser of FIG. 1 with a cap removed;
FIG. 10 is a perspective view showing an interior of the cap of the dispenser of FIG. 1;
FIG. 11 is a side cross-sectional view of the cap shown in FIG. 10;
FIG. 12 is a perspective view of a top portion of the dispenser of FIG. 1 with a housing of the cap removed but a seal of the cap shown transparent;
FIG. 12A is a detail view of the area labeled 12A in FIG. 2;
FIG. 13 is a cross-section view taken through the line 13-13 of FIG. 4;
FIG. 13A is a detail view of the area labeled 13A in FIG. 2;
FIG. 14 is a side cross-sectional view of another embodiment of dispenser in accordance with the invention described herein;
FIG. 14A is a detail view of the area labeled 14A in FIG. 14;
FIG. 14B is a perspective view of a plunger tip of the dispenser shown in FIG. 14;
FIG. 15 is a side cross-sectional view of another embodiment of dispenser in accordance with the invention described herein;
FIG. 15A is a detail view of the area labeled 15A in FIG. 15;
FIG. 15B is a perspective view of a plunger of the dispenser of FIG. 15;
FIG. 16A is a perspective view of an activation mechanism for an alternative embodiment of dispenser in accordance with the invention described herein; and
FIG. 16B is a cross-sectional view similar to FIG. 8A for the alternative embodiment of dispenser shown in FIG. 16A.

### DETAILED DESCRIPTION OF PREFERRED EMBODIMENT

The invention is directed to a dispenser that is configured to dispense a viscous fluid or concentrate. While the invention will be described in detail below with reference to various exemplary embodiments, it should be understood that the invention is not limited to the specific configuration or methodologies of any of these embodiments. In addition, although the exemplary embodiments are described as embodying several different inventive features, those skilled in the art will appreciate that any one of these features could be implemented without the others in accordance with the invention.

In this disclosure, references to "one embodiment," "an embodiment," "an exemplary embodiment," or "embodiments" mean that the feature or features being described are included in at least one embodiment of the invention. Separate references to "one embodiment," "an embodiment," "an exemplary embodiment," or "embodiments" in this description do not necessarily refer to the same embodiment and are also not mutually exclusive unless so stated and/or except as will be readily apparent to those skilled in the art from the description. For example, a feature, structure, function, etc. described in one embodiment may also be included in other embodiments, but is not necessarily included. Thus, the present invention can include a variety of combinations and/or integrations of the embodiments described herein.

As used herein, the term "nicotine" can be of plant origin or of synthetic or semi-synthetic origin. For example, it can be extracted from tobacco leaves or obtained by chemical synthesis. Nicotine may also refer to a nicotine substitute, which is typically a molecule that is not addictive but has a sensory effect similar to that of nicotine.

As used herein, the term "cannabis" refers to a genus of flowering plant in the family Cannabaceae. The number of species within the genus is disputed. Three species may be recognized, *Cannabis sativa, Cannabis indica* and *Cannabis ruderalis. C. ruderalis* may be included within C. sativa; or all three may be treated as subspecies of a single species, *C. sativa.* The genus is indigenous to central Asia and the Indian subcontinent.

Cannabis has long been used for hemp fiber, hemp oils, medicinal purposes, and as a recreational drug. Industrial hemp products are made from cannabis plants selected to produce an abundance of fiber. To satisfy the UN Narcotics Convention, some cannabis strains have been bred to produce minimal levels of tetrahydrocannabinol (THC), the principal psychoactive constituent. Many additional plants have been selectively bred to produce a maximum level of THC. Various compounds, including hashish and hash oil, may be extracted from the plant.

Within naturally occurring and manmade hybrids, cannabis contains a vast array of compounds. Three compound classes are of interest within the context of the present disclosure, although other compounds can be present or added to the compositions to optimize the experience of a given recreational consumer and medical or medicinal patient or patient population. Those classes include cannabinoids, terpenes and flavonoids.

There are many ways of growing cannabis, some of which are natural, and some are carefully designed by humans, and they will not be recited here. However, one of ordinary skill in the art of cannabis production will typically place a cannabis seed or cutting into a growth media such as soil, manufactured soil designed for cannabis growth or one of many hydroponic growth media. The cannabis seed or cutting is then provided with water, light and, optionally, a nutrient supplement. At times, the atmosphere and temperature are manipulated to aid in the growth process. Typically, the humidity, air to carbon dioxide gas ratio and elevated temperature, either by use of a heat source or waste heat produced by artificial light, are used. On many occasions ventilation is carefully controlled to maintain the conditions described above within an optimal range to both increase the rate of growth and, optionally, maximize the plant's production of the compounds, which comprise the compositions of the disclosure. It is possible to control lighting cycles to optimize various growth parameters of the plant.

Given the number of variables and the complex interaction of the variables, it is possible to develop highly specific formulas for production of cannabis which lead to a variety of desired plant characteristics. The present disclosure is applicable to use with such inventive means for growing cannabis as well as any of the variety of conventional methods.

*Cannabis sativa* is an annual herbaceous plant in the Cannabis genus. It is a member of a small, but diverse family of flowering plants of the Cannabaceae family. It has been cultivated throughout recorded history, used as a source of industrial fiber, seed oil, food, recreation, religious and spiritual moods and medicine. Each part of the plant is harvested differently, depending on the purpose of its use. The species was first classified by Carl Linnaeus in 1753.

*Cannabis indica,* formally known as *Cannabis sativa forma indica,* is an annual plant in the Cannabaceae family. A putative species of the genus Cannabis.

*Cannabis ruderalis* is a low-THC species of Cannabis, which is native to Central and Eastern Europe and Russia. It is widely debated as to whether C. *ruderalis* is a sub-species of *Cannabis sativa.* Many scholars accept *Cannabis ruderalis* as its own species due to its unique traits and phenotypes that distinguish it from *Cannabis indica* and *Cannabis sativa.*

As used herein, the term "cannabinoid" refers to a chemical compound belonging to a class of secondary compounds commonly found in plants of genus cannabis, but also encompasses synthetic and semi-synthetic cannabinoids.

The most notable cannabinoid is tetrahydrocannabinol (THC), the primary psychoactive compound in cannabis. Cannabidiol (CBD) is another cannabinoid that is a major constituent of the phytocannabinoids. There are at least 113 different cannabinoids isolated from cannabis, exhibiting varied effects.

Synthetic cannabinoids and semi-synthetic cannabinoids encompass a variety of distinct chemical classes, for example and without limitation: the classical cannabinoids structurally related to THC, the non-classical cannabinoids (cannabimimetics) including the aminoalkylindoles, 1,5 diarylpyrazoles, quinolines, and arylsulfonamides as well as eicosanoids related to endocannabinoids.

In many cases, a cannabinoid can be identified because its chemical name will include the text string "*cannabi*". However, there are a number of cannabinoids that do not use this nomenclature.

Within the context of this disclosure, where reference is made to a particular cannabinoid, each of the acid and/or decarboxylated forms are contemplated as both single molecules and mixtures. In addition, salts of cannabinoids are also encompassed, such as salts of cannabinoid carboxylic acids.

As well, any and all isomeric, enantiomeric, or optically active derivatives are also encompassed. In particular, where appropriate, reference to a particular cannabinoid incudes both the "A Form" and the "B Form". For example, it is known that THCA has two isomers, THCA-A in which the carboxylic acid group is in the 1 position between the hydroxyl group and the carbon chain (A Form) and THCA-B in which the carboxylic acid group is in the 3 position following the carbon chain (B Form).

Examples of cannabinoids include, but are not limited to: cannabigerolic acid (CBGA), cannabigerolic acid monomethylether (CBGAM), cannabigerol (CBG), cannabigerol monomethylether (CBGM), cannabigerovarinic acid (CBGVA), cannabigerovarin (CBGV), cannabichromenic Acid (CBCA), cannabichromene (CBC), cannabichromevarinic Acid (CBCVA), cannabichromevarin (CBCV), cannabidiolic acid (CBDA), cannabidiol (CBD), Δ6-cannabidiol (Δ6 CBD), cannabidiol monomethylether (CBDM), cannabidiol-C4 (CBD-C4), cannabidivarinic Acid (CBDVA), cannabidivarin (CBDV), cannabidiorcol (CBD-C1), tetrahydrocannabinolic acid A (THCA-A), tetrahydrocannabinolic acid B (THCA-B), tetrahydrocannabinol (THC or Δ9-THC), Δ8-tetrahydrocannabinol (Δ8-THC), *trans*-Δ10-tetrahydrocannabinol (*trans*-Δ10-THC), *cis*-Δ10-tetrahydrocannabinol (*cis*-Δ10-THC), tetrahydrocannabinolic acid C4 (THCA-C4), tetrahydrocannbinol C4 (THC C4), tetrahydrocannabivarinic acid (THCVA), tetrahydrocannabivarin (THCV), Δ8-tetrahydrocannabivarin (Δ8-THCV), Δ9-tetrahydrocannabivarin (Δ9-THCV), tetrahydrocannabiorcolic acid (THCA-C1), tetrahydrocannabiorcol (THC-C1), Δ7-cis-iso-tetrahydrocannabivarin, Δ8-tetrabydrocannabinolic acid (Δ8-THCA), Δ9-tetrabydrocannabinolic acid (Δ9-THCA), cannabicyclolic acid (CBLA), cannabicyclol (CBL), cannabicyclovarin (CBLV), cannabielsoic acid A (CBEA-A), cannabielsoic acid B (CBEA-B), cnnabielsoin (CBE), cannabinolic acid (CBNA), cannabinol (CBN), cannabinol methylether (CBNM), cannabinol-C4 (CBN-C4), cannabivarin (CBV), cannabino-C2 (CBN-C2), cannabiorcol (CBN-C1), cannabinodiol (CBND), cannabinodivarin (CBDV), cannabitriol (CBT), 11-hydroxy-Δ9-tetrahydrocannabinol (11-OH-THC), 11 nor 9-carboxy-δ9-tetrahydrocannabinol, ethoxy-cannabitriolvarin (CBTVE), 10 ethoxy-9-hydroxy-δ6a-tetrahydrocannabinol, cannabitriolvarin (CBTV), 8,9 dihydroxy-Δ6a(10a)-tetrahydrocannabinol (8,9-Di-OH-CBT-C5), dehydrocannabifuran (DCBF), cannbifuran (CBF), cannabichromanon (CBCN), cannabicitran (CBT), 10 oxo-Δ6a(10a)-tetrahydrocannabinol (OTHC), Δ9 cis tetrahydrocannabinol (cis THC), cannabiripsol (cbr), 3,4,5,6-tetrahydro-7-hydroxy-alpha-alpha-2-trimethyl-9-n-propyl-2,6-methano-2h-1-benzoxocin-5-methanol (OH-iso-HHCV), trihydroxy-delta-9-tetrahydrocannabinol (triOH-THC), yangonin, epigallocatechin gallate, dodeca-2e, 4e, 8z, 10z-tetraenoic acid isobutylamide, hexahydrocannibinol, and dodeca-2e,4e-dienoic acid isobutylamide.

In some embodiments of the present disclosure, the cannabinoid is a cannabinoid dimer. The cannabinoid may be a dimer of the same cannabinoid (e.g. THC-THC) or different cannabinoids. In an embodiment of the present disclosure, the cannabinoid may be a dimer of THC, including for example cannabisol.

As used herein, the term "cannabis concentrate" refers to a mixture of compounds that is obtained from a cannabis plant, such as for example a mixture of compounds or compositions that have been extracted from cannabis. The cannabis concentrate may be a concentrated composition of cannabis-derived cannabinoids, terpenes, terpenoids, and other naturally occurring compounds found in the cannabis plant. Non-limiting embodiments of a cannabis concentrate include a cannabis distillate, a cannabis isolate, a cannabis resin, a cannabis-derived cannabinoid, or any other type of extract containing one or more cannabinoids or terpenes, terpenoids, and other naturally occurring compounds found in the cannabis plant.

As used herein, the term "viscosity control agent" describes a substance for controlling and maintaining the viscosity of the payload. Non-limiting embodiments of a viscosity control agent include propylene glycol (1,2-propanediol), 1,3-propanediol, polyethylene glycol, vegetable glycerin, a terpene, triacetin, diacetin and triethyl citrate.

As used herein, the term "stabilizer" is any substance used to prevent an unwanted change in state. The stabilizer may be used to improve or maintain the stability of the payload. For example, without a stabilizer, cannabinoids or cannabis concentrates may be susceptible to degradation, such as oxidative degradation, cannabinoids may crystallize out of the payload, and/or the payload may undergo color change.

As used herein, the term "flavorant" is used to describe a compound or combination of compounds that may provide flavor and/or aroma to the payload. The flavorant may include at least one of a natural flavorant or an artificial flavorant. Non-limiting embodiments of a flavorant may be a tobacco flavor, menthol, wintergreen, peppermint, herb flavors, fruit flavors, nut flavors, liquor flavors and terpene flavors.

### First Exemplary Embodiment of Dispenser

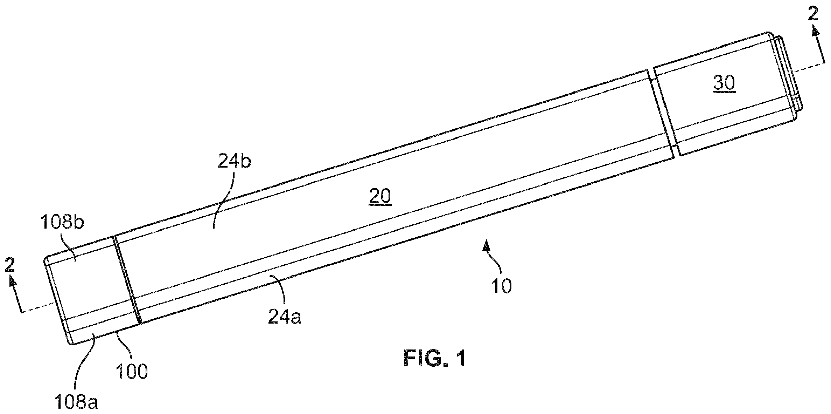

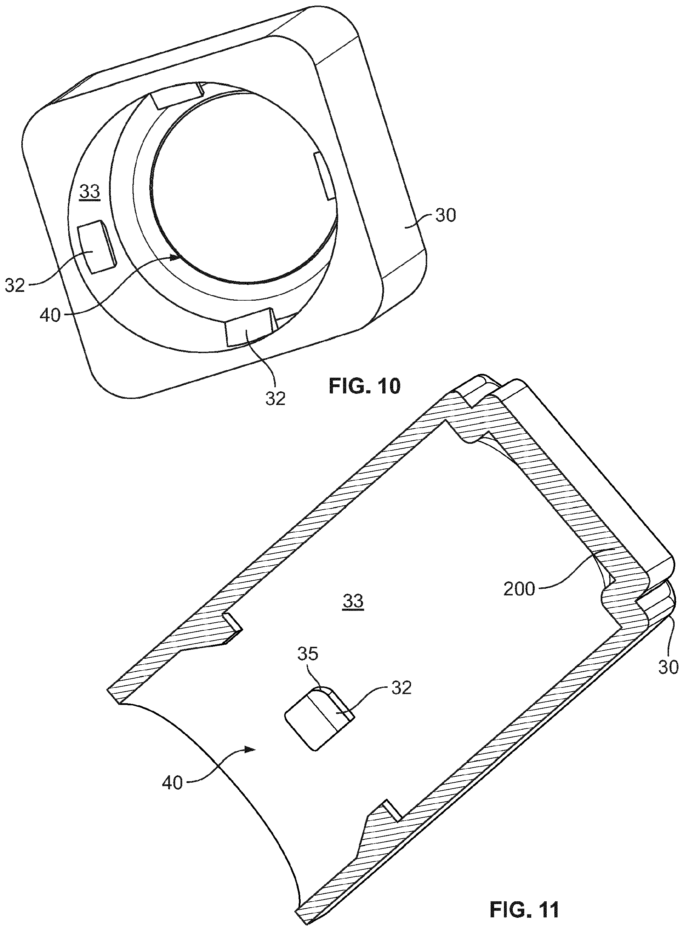

Referring to FIG. 1, a dispenser in accordance with one exemplary embodiment of the invention described herein is identified generally as 10. As described herein, dispenser 10 may be configured for dispensing controlled amounts of a viscous fluid or concentrate and providing feedback to the user so that the user knows when a certain amount of the viscous fluid or concentrate has been dispensed. As depicted in FIG. 1, dispenser 10 may include a housing 20 and a cap 30 releasably connectable to housing 20. Such connection may be sealable to inhibit an escape of vapor from an interior 40 (FIGS. 10-11) of cap 30. The viscous fluid or concentrate may include nicotine, cannabis, a cannabinoid or a cannabis concentrate as an ingredient. The viscous fluid or concentrate may further include other components such as, without limitation, a viscosity control agent, a stabilizer, and a flavorant.

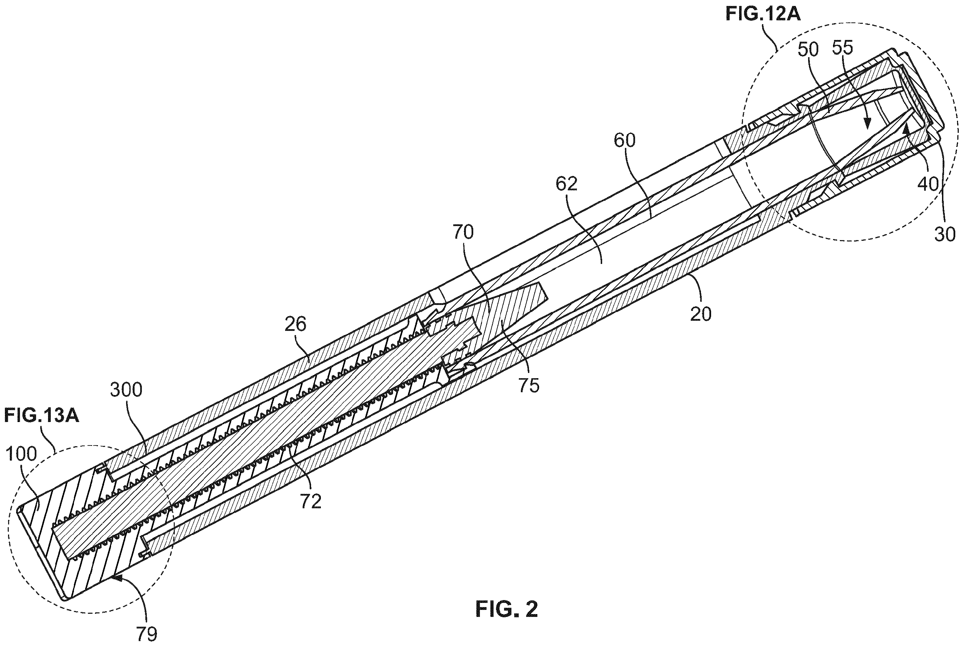

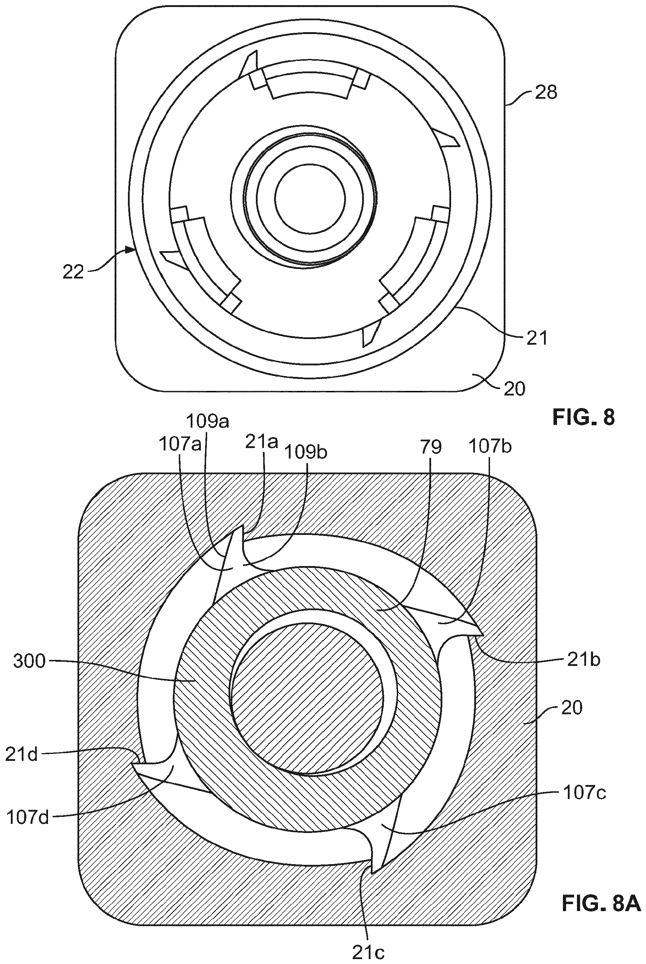

The housing 20 includes an outer casing 26 (FIG. 2) and a barrel 60 positioned within a cavity of the outer casing 26. The outer casing 26 has an inner surface 21 bounding a cylindrical inner cavity 22 as shown in FIG. 8. The outer casing 26 has an external surface 28 that has a generally square cross-section. The barrel 60 defines a reservoir 62 (FIG. 2) that is configured to retain a viscous fluid or concentrate. The barrel 60 may be coupled to outer casing 26 in a manner that securely retains the barrel 60 within the outer casing 26 and prevents rotation and axial movement of the barrel 60 with respect to the outer casing 26 (e.g., a key on the barrel 60 or outer casing 26 may be retained within a keyway of the other of the barrel 60 or outer casing 26).

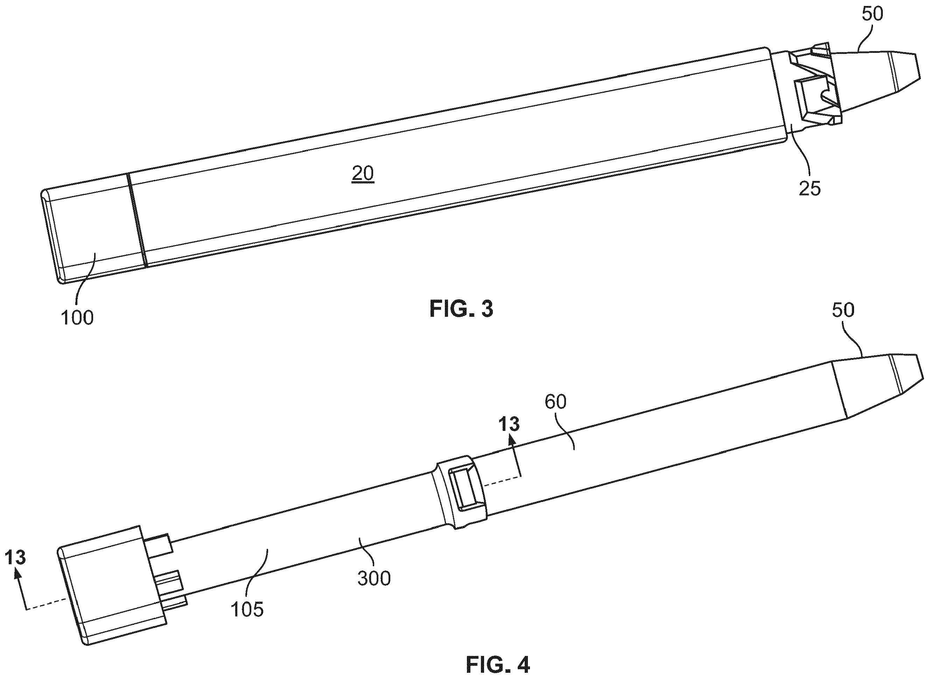

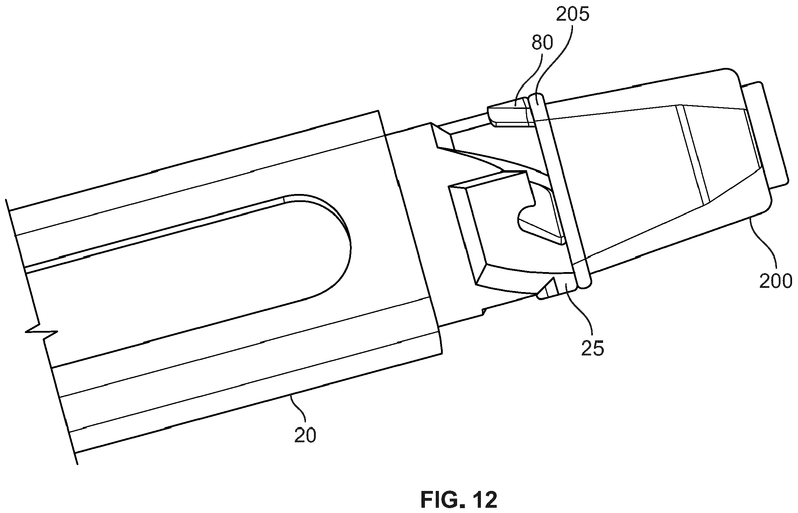

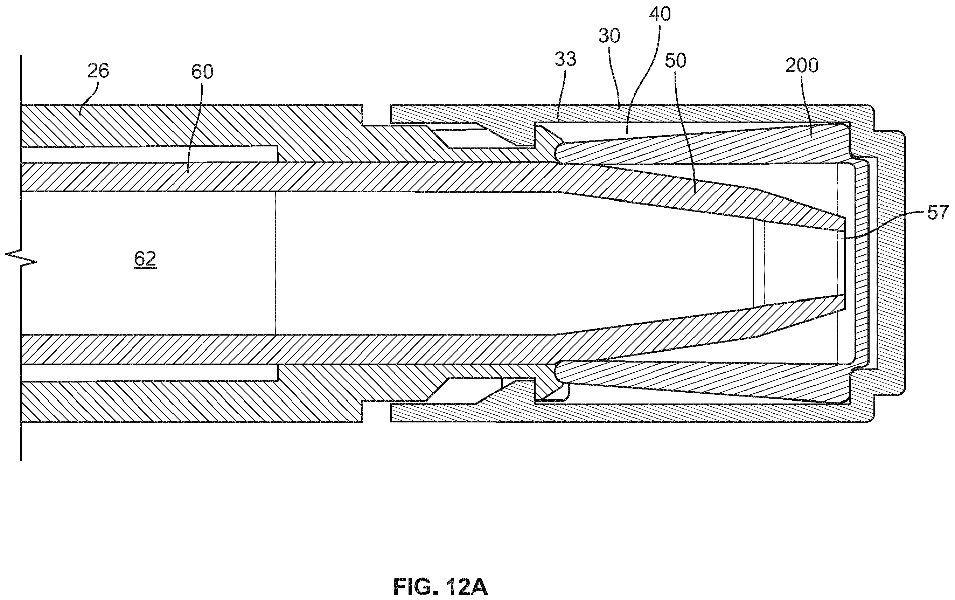

As depicted in cross-section in FIG. 12A, barrel 60 includes a tip 50 that extends away from the end of outer casing 26 to be received in the interior 40 of cap 30 when cap 30 is connected to housing 20. As depicted in FIG. 3, tip 50 may extend axially from the outer casing 26 of housing 20. Tip 50 may be hollow and formed of a high-temperature resistant material such as tempered glass, stainless steel, titanium or any other alloy that can withstand the temperatures encountered in vaporization rigs. Alternatively, the tip 50 may be made of other refractory materials such a quartz, alumina ceramic or other heat resistant glass, mineral or composite. In order to accommodate various viscosities of concentrates, a length and an inner diameter of a tip cavity 55 (FIG. 2) of tip 50 may be varied to prevent unintended oozing of concentrate from dispenser 10. Tip cavity 55 is in fluid communication with the reservoir 62, and tip cavity 55 ends at a dispensing orifice 57 (FIG. 12A) through which the fluid within reservoir 62 may be dispensed. The diameter of the dispensing orifice 57 may be modified depending on the type of fluid held within the reservoir 62. For example, for fluids having a higher viscosity, the diameter of the dispensing orifice 57 may be larger than for fluids having a lower viscosity.

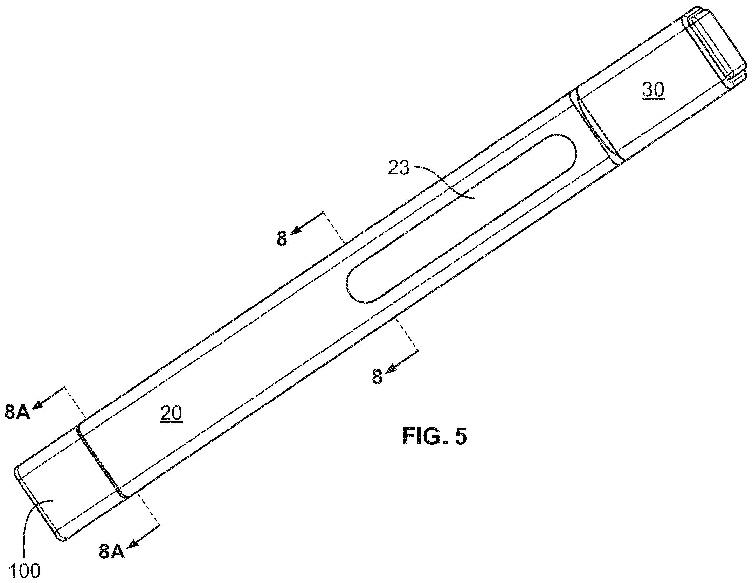

Barrel 60 may be made of any food safe plastic, metal or glass. Barrel 60 may also be formed from any of the materials specified above for tip 50. Tip 50 may be integrally formed with the remainder of barrel 60 or formed separately from the remainder of barrel 60 and connected to the remainder of barrel 60. Preferably, barrel 60 is constructed of a transparent material to allow the user to inspect the amount of concentrate remaining in the reservoir 62. In an example, housing 20 may include a transparent window 23 (FIG. 5) to allow a user to view barrel 60 therethrough and inspect the amount of concentrate remaining in reservoir 62. The window 23 can be made of polystyrene or glass, or any other suitable transparent material.

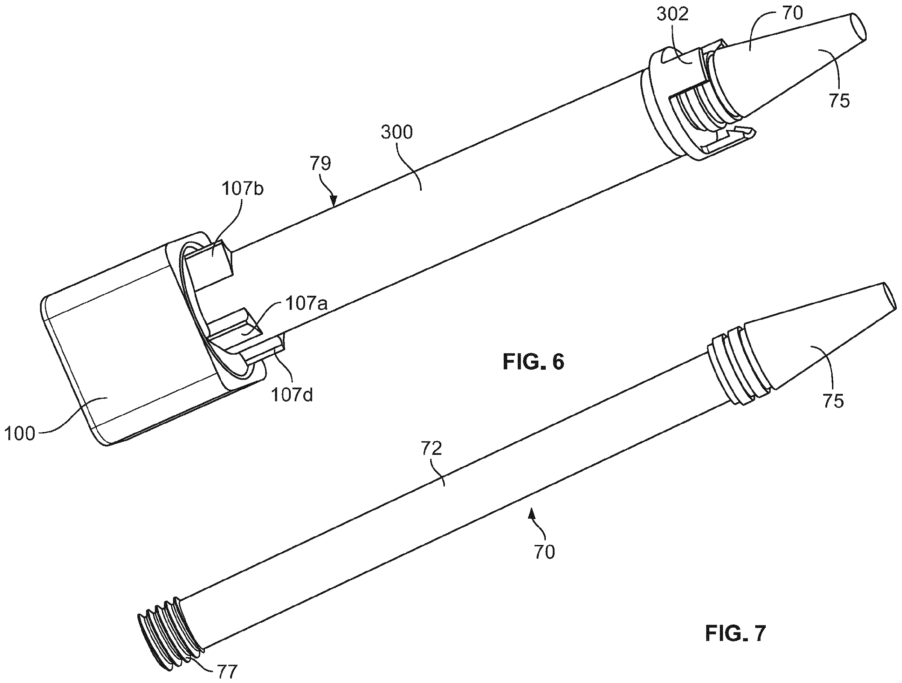

A piston or plunger 70 may be located within housing 20 and barrel 60 as depicted in FIG. 2, for example. Referring to FIG. 7, plunger 70 includes a shaft 72 with threads 77 at one end thereof and a plunger tip 75 at an opposite end. The plunger tip 75 may include an internal cavity that receives an end of shaft 72. The plunger tip 75 may be frictionally retained on the end of shaft 72. The plunger tip 75 may be made from a food-safe elastomer such as silicone rubber or LDPE. The plunger tip 75 may have a complementary shape to tip cavity 55 of tip 50 as to minimize any holdup as the last of the concentrate is ejected, i.e., when plunger 70 has advanced from the position shown in FIG. 2 through the reservoir 62 to tip cavity 55 during dispensing of a liquid in barrel 60. The threaded shaft 72 may be formed of metal or plastic.

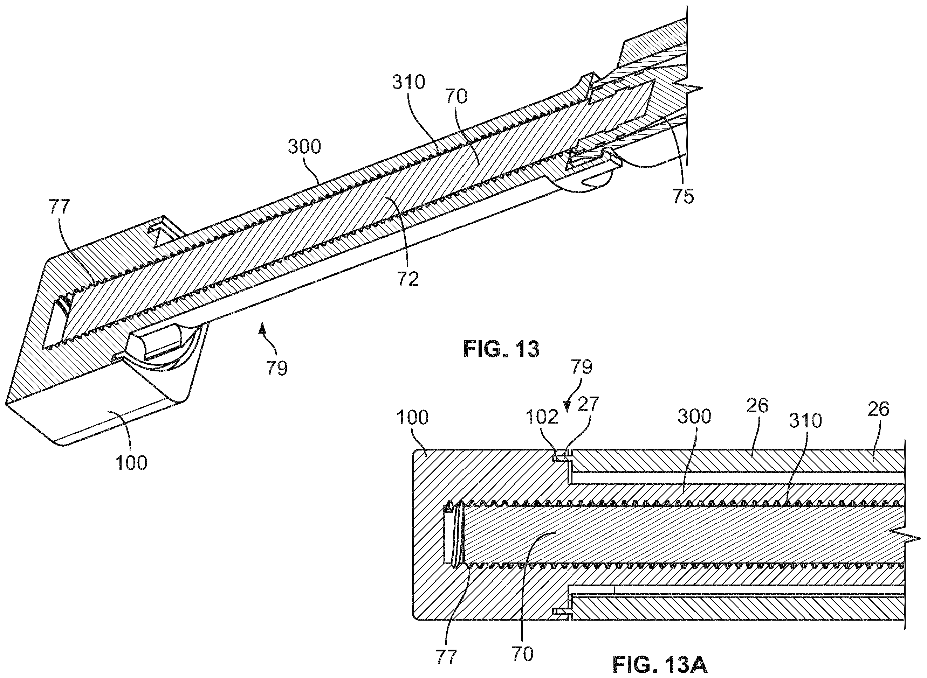

An activation mechanism 79 is coupled to plunger 70 for moving plunger 70 from the position shown in FIG. 2 through the reservoir 62 to tip cavity 55. The activation mechanism 79 includes a knob 100 and a sleeve 300. As shown in FIGS. 13 and 13A, the threads 77 on plunger 70 engage an interior threaded surface 310 of activation mechanism 79. The interior threaded surface 310 extends from the knob 100 at one end of activation mechanism 79 through the sleeve 300 to the other end of activation mechanism 79. Activation mechanism 79 imparts an axial force on plunger 70 when activation mechanism 79 is rotated with respect to plunger 70 such that plunger advances axially toward tip 50 to dispense liquid from dispensing orifice 57. Threaded sleeve 300 is located in housing 20, and knob 100 is positioned outside of the housing 20. Sleeve 300 is attached to knob 100, which provides a gripping surface for applying a twisting motion to cause rotation of interior threaded surface 310 to provide the axial force to plunger 70 described above. Plunger 70 may be rotationally fixed with respect to activation mechanism 79 by for example frictional engagement between plunger 70 and the inner wall of barrel 60, or a key or keyway on plunger 70 that engages a keyway or key formed on barrel 60.

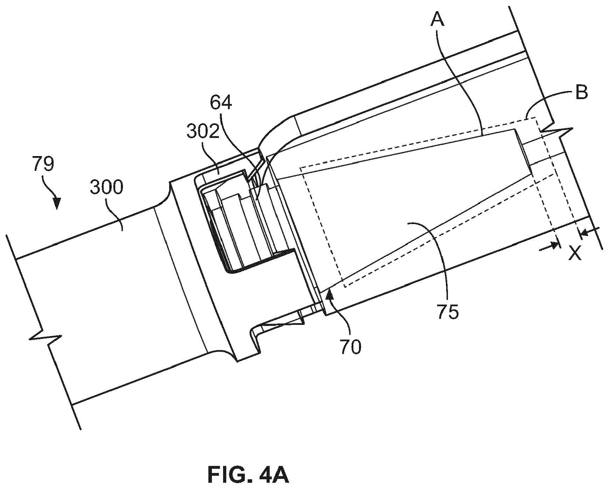

As shown in FIG. 13A, an annular recess 102 formed in knob 100 receives an annular projection 27 of housing 20. The recess 102 and projection 27 allow knob 100 to rotate with respect to housing 20 and maintain knob 100 in a desired position with respect to housing 20. Referring to FIG. 4A, the end of sleeve 300 opposite knob 100 includes clips, one of which is identified as 302. The clips 302 are received within a recess 64 formed in an end of barrel 60 to couple the activation mechanism 79 to the barrel 60. The recess 64 extends around the circumference of barrel 60 to allow the activation mechanism 79 to rotate with respect to barrel 60 as knob 100 is rotated by a user. The connection between the activation mechanism 79 and the barrel 60 retains the activation mechanism 79 axially with respect to housing 20. Other connections between the activation mechanism 79 and barrel 60 that axially retain the activation mechanism 79 to the barrel 60 while allowing rotational movement of the activation mechanism 79 with respect to the barrel 60 are within the scope of the invention.

Activation mechanism 79 and housing 20 are configured to provide feedback to a user when the user rotates the activation mechanism 79 in a direction that advances plunger 70 toward dispensing orifice 57. Activation mechanism 79 includes four teeth 107a-d, shown in FIG. 8A, that extend radially outward from the exterior surface of sleeve 300. The teeth 107a-d are spaced equidistant from each other around the circumference of sleeve 300. The teeth 107a-d have a substantially similar structure. Accordingly, only tooth 107a is described in detail herein. Tooth 107a has an angled surface 109a that extends tangentially outward from the exterior surface of sleeve 300, and a curved surface 109b that extends between an end of the angled surface 109a back to the exterior surface of sleeve 300.

The inner surface 21 of housing 20 includes four engaging surfaces 21a-d spaced equidistant around the circumference of inner surface 21. As shown in FIG. 8A, each of the engaging surfaces 21a-d is configured to simultaneously engage one of the teeth 107a-d to arrest rotation of activation mechanism 79 relative to housing 20 in the clockwise direction when viewed as shown in FIG. 8A. As shown in FIG. 8A, when tooth 107a engages engaging surface 21a, tooth 107b engages surface 21b, tooth 107c engages surface 21c, and tooth 107d engages surface 21d. Activation mechanism 79 may be rotated by a user in the clockwise direction shown in FIG. 8A so that the teeth 107a-d move past the engaging surfaces 21a-d. For example, the teeth 107a-d and/or the engaging surfaces 21a-d may slightly deflect until there is enough clearance for the teeth 107a-d to move past the engaging surfaces 21a-d. As the teeth 107a-d move past the engaging surfaces 21a-d, tactile feedback is provided to the user in that an increased amount of torque is require to move the teeth 107a-d past the engaging surfaces 21a-d than the amount of torque required to rotate the activation mechanism 79 when the teeth 107a-d are between adjacent engaging surfaces 21a-d. Audible feedback may also be provided to the user when the teeth 107a-d move past the engaging surfaces 21a-d as the deflection of the teeth 107a-d and/or engaging surfaces 21a-d may cause a snapping or clicking sound.

The tactile and/or audible feedback may be utilized by a user to inform the user approximately how much concentrate is dispensed through dispensing orifice 57. For example, if the user begins to use dispenser 10 when it is in the position shown in FIG. 8A, the user removes the cap 30, grasps the knob 100, and rotates the activation mechanism 79 clockwise when viewed as shown in FIG. 8A. As the activation mechanism 79 rotates, interior threaded surface 310 engages the threads 77 of plunger 70 to move plunger 70 axially in a direction toward dispensing orifice 57. As activation mechanism 79 rotates, plunger 70 is constrained from rotation by engagement with barrel 60 thereby causing axial movement of plunger 70. When the user rotates activation mechanism 79 approximately 90 degrees clockwise from the position shown in FIG. 8A, tooth 107a will engage surface 21b, tooth 107b will engage surface 21c, tooth 107c will engage surface 21d, and tooth 107d will engage surface 21a. At this point, the user will feel the engagement between the teeth 107a-d and surfaces 21a-d in the form of resistance to rotation of knob 100. This tactile feedback alerts the user to the fact that the activation mechanism 79 has rotated approximately 90 degrees. The ninety degree rotation of activation mechanism 79 correlates to an axial distance X (FIG. 4A) that plunger has advanced into reservoir 62 toward dispensing orifice 57 (e.g., plunger 70 may move from the position A identified in FIG. 4A to the position B identified in FIG. 4A due to ninety degree rotation of activation mechanism 79). As known, this axial distance may be determined based on the thread pitch of threads 77 and interior threaded surface 310. The axial distance X further correlates to a volume of concentrate that has been displaced from reservoir 62 and forced to dispense through dispensing orifice 57. As known, this volume may be determined based on the radius of the reservoir 62 and the axial distance X. Instructions may be provided with dispenser 10 to inform a user of the volume of concentrate dispensed for each ninety degree rotation of activation mechanism 79. Thus, by utilizing the tactile feedback provided by the teeth 107a-d and engaging surfaces 21a-d and knowing how much concentrate is dispensed for each ninety degree rotation of activation mechanism 79, a user may know and control the amount of concentrate that is dispensed from dispenser 10 just by rotating the knob 100.

Instead of the teeth 107a-d and engaging surfaces 21a-d, dispenser 10 may be modified to include any type of detent mechanism that is designed to provide feedback to the user when activation mechanism 79 has been rotated a certain number of degrees. For example, dispenser 10 may have a detent mechanism using metal springs and ball bearings, or pawls sprung on metal or plastic tabs. Alternatively, any suitable mechanism that gives either an aural or haptic feedback on the progress (i.e., degree of rotation) of the twisting motion between housing 20 and knob 100 during advancement of plunger 70 may be used.

Housing 20 and knob 100 are oriented so that when teeth 107a-d engage surfaces 21a-d, the outer surfaces of housing 20 and knob 100 are aligned. For example, when teeth 107a-d are in the position shown in FIG. 8A, housing 20 and knob 100 are in the positions shown in FIG. 1, in which exterior surfaces 24a-b of housing 20 are aligned with exterior surfaces 108a-b of knob 100 (and two additional exterior surfaces of housing 20 and knob 100 on the other side of dispenser 10 are also aligned). When knob 100 is rotated 90 degrees clockwise from the position shown in FIG. 8A, exterior surface 108a of knob 100 is aligned with exterior surface 24b of housing 20, and the other three exterior surfaces of knob 100 are aligned with exterior surfaces of housing 20. It is within the scope of the invention for knob 100 and housing 20 to have different shapes (e.g., each may be cylindrical or have a triangular cross-section or any other polygonal cross-section). Further, it is within the scope of the invention for there to be more or less than four teeth 107a-d and engaging surfaces 21a-d. For example, if there is only one tooth 107a and one engaging surface 21a, knob 100 would rotate 360 degrees before the user receives feedback in the nature of increased resistance to rotation. The dispenser 10 may also be configured so that knob 100 rotates any other number of degrees before the user receives feedback (e.g., 180 degrees or 270 degrees).

As described above, for the embodiment of dispenser 10 shown in the drawings, each rotation of knob 100 of about ninety degrees may cause a desired dose of cannabis concentrate to be dispensed from dispensing orifice 57. For example, a consistent dose may be dispensed for each ninety degree turn for use on a hot dab ring crown, for oral ingestion, to add as an active ingredient to edibles or for use in filling a vape cartridge.

Housing 20 and knob 100 may have a square cross-section shape as depicted. Alternatively, housing 20 and/or the knob 100 may be cylindrical. In such a case, the plunger 70 and activation mechanism 79 may be configured so that threads 77 and interior threaded surface 310 are formed from low friction materials so that a user can readily apply the amount of torque necessary to advance the plunger 70 through reservoir 62. Further, the teeth 107a-d and engaging surfaces 21a-d may be configured so that a user is able to apply the amount of torque necessary to move the teeth 107a-d past the engaging surfaces 21a-d while still receiving suitable feedback informing the user that the teeth 107a-d have advanced to a position where they engage the engaging surfaces 21a-d. The shape of dispenser 10 may be easily distinguishable from a syringe, and the cap 30 may allow dispenser 10 to be safely stored in a pocket or bag. The cap 30 can optionally be equipped with a child-resistant closure as described below to prevent children from accessing the material (e.g., liquid in reservoir 62).

The housing 20 can be made from plastic such as ABS, polystyrene, or any other suitable plastic, or, can be made from wood or metal.

Dispenser 10 may typically hold in reservoir 62 from about 0.5g to about 1g or from about 0.1g to about 0.5g of concentrate.

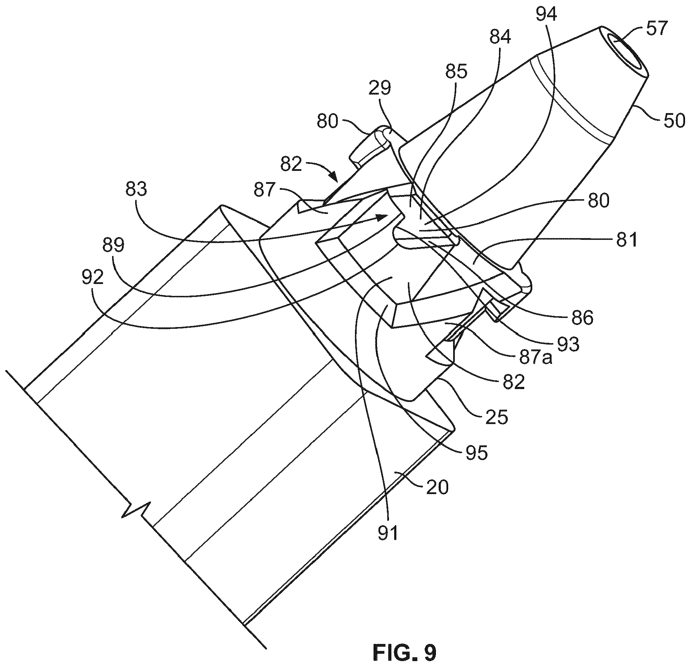

As depicted in FIGS. 3 and 9, a top extending portion 25 of housing 20 is positioned at an end of housing 20 opposite knob 100 with tip 50 extending outward from extending portion 25. Top extending portion 25 may include connecting members 80 configured (e.g., shaped and dimensioned) to connect housing 20 to cap 30. Cap 30 may include engaging members 32 (FIGS. 10 & 11) configured (e.g., shaped and dimensioned) to engage connecting members 80 to connect cap 30 to housing 20.

Connecting members 80 may be bayonet shaped, for example, as depicted in FIGS. 3 and 9 while engaging members 32 (FIGS. 10 & 11) may project from an inner surface 33 of cap 30 and may be cube shaped except for ramped portions 35 at a top corner thereof. Connecting members 80 may project from adjacent recessed surfaces 82.

Cap 30 may be located on extending portion 25 such that an interior 40 of cap 30 receives extending portion 25 and tip 50. Cap 30 may be moved longitudinally toward knob 100 such that engaging members 32 may be moved longitudinally via an entry recess 81 defined by recessed surfaces 82. Cap 30 may be rotated clockwise via a circumferentially extending recess 91 defined by adjacent recessed surfaces 82 such that one of engaging members 32 may be located in a holding recess 83 bounded by a tooth 84 of one of connecting members 80. The entry recess 81 extends from a top end 29 of the housing 20 to the circumferentially extending recess 91 and the holding recess 83. The entry recess 81 is defined by the tooth 84, an axially extending portion 87a of an adjacent connecting member, and a bottom circumferentially extending recess bounding portion 95. Tooth 84 may extend longitudinally (i.e., axially relative to an axial dimension of housing 20) from a circumferentially extending portion 85 of one of connecting members 80. Holding recess 83 may also be bounded by extending portion 85 and a triangularly shaped axially extending portion 87. Holding recess 83 may be one of a plurality (e.g., 4) of holding recesses around a circumference of extending portion 25.

Cap 30 may include a seal 200 (FIGS. 12A) in interior 40 of cap 30 bounded by inner surface 33. Seal 200 may be formed of low density polyethylene (LDPE) for example and may be elastically deformable such that seal 200 may be compressed longitudinally (i.e., axially relative to housing 20) by a user relative to dispenser 10 when the user presses on cap 30 in a longitudinal direction toward knob 100. Seal 200 may be biased such that when such pressure is released seal 200 tends to return in a direction away from knob 100. For example, seal 200 may include a circumferential bottom portion 205 (FIG. 12) that may contact a top end 29 (FIG. 9) of extending portion 25 and may be resiliently compressed by an axial force (e.g. by a user pressing on cap 30) such that a bias force may be provided in the opposite direction (i.e., away from knob 100). Seal 200 may sealingly engage the top end 29 to provide a seal thereby inhibiting fluid communication between interior 40 of cap 30 and reservoir 62 of housing 20 relative to the ambient environment.

To connect cap 30 to top extending portion 25 of housing 20 to close dispenser 10, cap 30 may be pressed longitudinally (i.e., axially) by a user relative to dispenser 10 to allow one of engaging members 32 to move axially past tooth 84 and in a circumferential direction as cap 30 is rotated. For example, a force of 5 kgf (kilogram-force) may be required in the longitudinal direction (relative to dispenser 10) to overcome a resiliency of seal 200 to allow one of the engaging members 32 to move past tooth 84. Pressure on cap 30 may be released such that seal 200 may expand in a direction away from knob 100 due to a resilient bias of seal 200 as described above. Thus, in response to the release of pressure and resiliency of seal 200, cap 30 and engaging members 32 may move longitudinally (i.e., axially) relative to dispenser 10 away from knob 100 and one of such engaging members 32 may be received in holding recess 83 and such engaging member 32 may be blocked from being rotated counterclockwise by tooth 84 absent pressure being applied to cap 30. Multiple such engaging members 32 may be received in multiple holding recesses 83 to inhibit movement of cap 30 in a counterclockwise direction. The release of the cap 30 and resilient bias of seal 200 may provide a force in the direction away from knob 100 to bias engaging members 32 within the holding recesses 83 and against bottom surfaces 89 of extending portion 85.

To open dispenser 10 when the engaging members 32 are received within the holding recesses 83, pressure may be applied to cap 30 by a user longitudinally (i.e., axially) relative to dispenser 10 toward knob 100 compressing seal 200 such that engaging members 32 may extend longitudinally past connecting members 80 and cap 30 may be rotated counterclockwise until the engaging members 32 have moved circumferentially (e.g., in recess 91) past connecting members 80. The pressure may then be released and the engaging members 32 may pass through a recess (e.g., entry recess 81) to allow cap 30 to be removed from top extending portion 25 of housing 20.

In an example, a user may apply axial and rotational pressure to engage cap 30 with extending portion 25 by engaging engaging members 32 with the holding recesses (e.g, holding recess 83) using one hand. In another example, a user may apply axial and rotational pressure to disengage cap 30 from extending portion 25 by disengaging engaging members 32 from the holding recesses (e.g., holding recess 83) using one hand.

In addition, seal 200 may be vapor proof when cap 30 is engaged with extending portion 25. Specifically, a resiliency force of seal 200 biasing cap 30 away from extending portion 25 may hold engaging members 32 within the holding recesses 83. The bias force holding engaging members 32 within the holding recesses may be balanced with the force required to move cap longitudinally to disengage engaging members 32 to allow an opening of dispenser 10, including the frictional characteristics (e.g., static and dynamic friction) of the cap 30 and extending portion 25 that opposes the rotational motion, such that a required axial force and rotational friction does not make the twisting motion too difficult for elderly persons.

In an example, connecting members 80 may include ramped sides 86 and engaging members 32 may move along the ramped sides during clockwise rotation of cap 30 to facilitate entry of one such engaging members (e.g., through recess 81 and recess 91) into holding recess 83 and other such engaging members into similar or identical such holding recesses. Ramped sides 86 may be aligned at about a 45 degree angle relative to a longitudinal axis of dispenser 10. Ramped portions 35 of engaging members 32 may similarly facilitate movement of the engaging members in a counterclockwise direction along connecting members 80 after cap 30 has been pressed toward knob 100. Ramped portions 35 may be aligned at about a 45 degree angle relative to a longitudinal axis of dispenser 10.

Referring to FIG. 9, the tooth 84 may include ramped side 86, a circumferentially extending side 92, and an axially extending side 93. The axially extending side 93 connects to the bottom surface 89 of extending portion 85. Each of the ramped side 86, the circumferentially extending side 92, and the axially extending side 93 may be a curved surface transitioning to a top surface 94 of the tooth 84.

As depicted in the figures, cap 30 and housing 20 may have similar or identical outside cross-sectional sizes and shapes. As depicted housing 20 and cap 30 may have identical or similar outside cross-sectional square shapes. In other examples, housing 20 and cap 30 could have identical or similar outside rectangular, circular or oval shapes. In further examples, housing 20 and cap 30 could have identical or similar outside cross-sectional shapes of other polynomials.

The described similar or identical outside cross-sectional sizes and shapes of cap 30 and housing 20 allow a user to easily ascertain if a closure mechanism of dispenser 10 is properly engaged. More specifically, the similar or identical cross-sectional sizes and shapes make it easier for a user to identify the alignment of surfaces of cap 30 and housing 20 (e.g., by touch or sight) when the cap 30 and extending portion 25 are engaged such that engaging members 32 are received in the holding recesses 83. In contrast, if the shapes of the cap and housing 20 differed, e.g., if either the cap is round and the body square, or the cap is square and the body is round, it would be more difficult to ascertain if such a closure mechanism is properly engaged (i.e., engaging members 32 are received in the holding recesses). Further, differences between a child's manual dexterity and cognitive abilities versus that of an elderly adult may allow an adult to easily open dispenser 10 when the closure mechanism is properly engaged (i.e., engaging members 32 are received in the holding recesses) while being difficult if not impossible for children to operate.

Importantly, an ultimate movement required to rotate the cap (e.g., cap 30) after applying axial pressure is the torque required to rotate the cap (e.g., clockwise to locate engaging members 32 in the holding recesses) which is a function of the friction of the rotating cap relative to housing 20, as well as its diameter. It would be understood by one skilled in the art that it would be more difficult to apply a given torque to a cap with a small diameter (or dimension) as opposed to a cap with a large diameter (or dimension). For example, there would be less surface area to apply a force to, and less leverage available for such force, with a smaller cap. It would also be understood by one skilled in the art that when a diameter of a container body (e.g., housing 20) is wider than a diameter of a cap (e.g., cap 30), a cognitive signal is presented that a twisting motion may undo the cap, in analogy with the common non-child resistant containers such as soda bottles and mayonnaise jars.

One of the characteristic differences between adults and children is hand size, and thus a thickness (if square) or diameter (if round) of a cap (e.g., cap 30), and a housing (e.g., housing 20) are critical to differentiating child and adult ease of opening. Further, a diameter or thickness of a cap (e.g., cap 30), and a housing (e.g., housing 20) may be manipulated to differentiate child and adult ease of opening since the absolute dimensions are critical to the ease of gripping such a housing and cap, and applying an axial force and simultaneously applying a torque force.

As described above, when cap 30 is engaged with extending portion 25, engaging members 32 may be received in holding recesses 83. In one example, after an axial force is applied to move the engaging members 32 axially or longitudinally closer to the knob 100 than tooth 84 (or a similar tooth or structure) a rotation of 45 degrees may be required to disengage the mechanism, i.e., to move the tooth circumferentially such that one or more of engaging members 32 may move axially or longitudinally in a direction toward a top end 29 of housing 20 due to a resilient force of seal 200 or a force applied by a user. The amount of rotation required would be dependent on a number, a size and a placement of connecting members 80. In another example, a rotation of 135 degrees may be required to disengage such a mechanism. In a further example, a rotation of 225 degrees may be required to disengage the mechanism. In yet another example, a rotation of 315 degrees may be required to disengage the mechanism. In yet a further example, any rotation other than 0 to 5 degrees or 355 to 360 degrees (i.e., a rotation between 5 to 355 degrees) may be required to engage or disengage the mechanism if the cross section of the body and cap is oval. In one aspect, any rotation other than 360/n degrees may be required to engage or disengage the mechanism if the cross section of the body and cap is an n sided polygon.

As suggested above, cross-sections of a cap (e.g., cap 30) and a body (e.g., housing 20) may be aligned when a closing mechanism of a container (e.g., dispenser 10) is in an engaged and locked state (e.g., when engaging members 32 may be received in holding recesses 83 of extending portion 25). In another example, cross-sections of a cap (e.g., cap 30) and a body (e.g., housing 20) may not be aligned when the closing mechanism is in the disengaged and unlocked state.

As indicated above, in an example, it may be visually apparent (e.g., cross-sections of a cap and a body or casing may be aligned) when a closing mechanism of a container (e.g., dispenser 10) is in an engaged and locked state. In another example, it may be visually apparent when the mechanism is in the disengaged and unlocked state.

As indicated above, in an example, it may be tactilely apparent (e.g., cross-sections of a cap and a body or casing may be aligned) when a closing mechanism of a container (e.g., dispenser 10) is in an engaged and locked state. In another example, it may be tactilely apparent when the mechanism is in the disengaged and unlocked state.

In an example, a thickest side (if square or rectangular in cross-section) or diameter (if round in cross-section) of a cap (e.g., cap 30) and a body (e.g., housing 20) may be between 0.5 and 1.25 inches, between 0.65 and 1 inches, or between 0.7 and 0.8 inches.

In an example, a total length of an engaged and locked cap and body (e.g., dispenser 10 when cap 30 is engaged with extending portion 25) may be between about 2 and about 6 inches. In another example, an engaged and locked cap and body (e.g., dispenser 10 when cap 30 is engaged with extending portion 25) may be between about 3 and about 5 inches.

In an example, cross-sections of a cap (e.g., cap 30) and a body (e.g., housing 20) may have square external cross-sections. In another example, cross-sections of a cap (e.g., cap 30) and a body (e.g., housing 20) may have round internal cross-sections. In a further example, cross-sections of a cap (e.g., cap 30) and a body (e.g., housing 20) may have square internal cross-sections. In yet another example, cross-sections of a cap (e.g., cap 30) and a body (e.g., housing 20) may have oval external cross-sections. In yet a further example, cross-sections of a cap (e.g., cap 30) and a body (e.g., housing 20) may have rectangular internal cross-sections. In an example, cross-sections of a cap (e.g., cap 30) and a body (e.g., housing 20) may have rectangular external cross-sections. In another example, cross-sections of a cap (e.g., cap 30) and a body (e.g., housing 20) may have polygonal internal cross-sections. In a further example, cross-sections of a cap (e.g., cap 30) and a body (e.g., housing 20) may have polygonal external cross-sections.

In one aspect, a container (e.g., dispenser 10) may be configured such that a shape and dimensions thereof (e.g., external dimensions of a cap and casing) are small relative to the hands of an adult so that a torque required to rotate a depressed cap (e.g., cap 30) from a locked (e.g., when engaging members are received in holding recesses) to an unlocked position (e.g., when engaging members are released from holding recesses) is about 0.791 Nm (Newton-metre).

In an example an axial force (i.e., in a direction toward knob 100) to release a cap (e.g., cap 30) from a housing (e.g., extending portion 25 connected to housing 20) by pressing engaging members (engaging members 32) of the cap axially past any retaining members (e.g., tooth 84) to overcome any resilient force (e.g., from a seal) may range from about 2 kgf to 8 kgf. In another example, such an axial force to overcome the resilient force and to press engaging members (engaging members 32) of the cap axially past any retaining members (e.g., tooth 84) may range from about 3 kgf to 7 kgf. In a further example, an axial force to overcome the resilient force and to press engaging members (engaging members 32) of the cap axially past any retaining members (e.g., tooth 84) may range from about 4 kgf to 6 kgf.

In an example, an axial force to overcome the resilient force and to press engaging members (engaging members 32) of the cap axially past any retaining members (e.g., tooth 84) may be about 2 kgf. In another example, an axial force to overcome the resilient force and to press engaging members (engaging members 32) of the cap axially past any retaining members (e.g., tooth 84) may be about 3 kgf. In yet another an example, an axial force to overcome the resilient force and to press engaging members (engaging members 32) of the cap axially past any retaining members (e.g., tooth 84) may be about 4 kgf. In a further example, an axial force to overcome the resilient force and to press engaging members (engaging members 32) of the cap axially past any retaining members (e.g., tooth 84) may be about 5 kgf. In yet a further example, an axial force to overcome the resilient force and to press engaging members (engaging members 32) of the cap axially past any retaining members (e.g., tooth 84) may be about 6 kgf. In an example, an axial force to overcome the resilient force and to press engaging members (engaging members 32) of the cap axially past any retaining members (e.g., tooth 84) may be about 7 kgf. In another example, an axial force to overcome the resilient force and to press engaging members (engaging members 32) of the cap axially past any retaining members (e.g., tooth 84) may be about 8 kgf.

As indicated above, an axial force may be applied to a cap (e.g., cap 30) in a direction toward knob 100 to press engaging members (engaging members 32) of the cap axially past any retaining members (e.g., tooth 84) to overcome any resilient force (e.g., from a seal) toward the free end of the cap. After such engaging members are located axially past any retaining members, the cap may be rotated (e.g., counter clockwise) to allow the engaging members to be located in circumferential spaces (e.g., recesses 81, 91) between the retaining members (e.g., connecting members 80) and the cap may then be moved away from knob 100 to release the cap from the housing 20. Such rotation of the cap may require a torque of about 0.1 Nm to about 1 Nm. In another example, the required torque may be about 0.1 Nm to about 0.9 Nm. In a further example the torque required to rotate the cap to allow the engaging members to be located in circumferential spaces between the retaining members (e.g., tooth 84) and the cap may be less than or equal to about 0.791 Nm. In another example, the required torque may be about 0.791 Nm.

As described above, extending portion 25 of housing 20 may include connecting members 80 bounding holding recesses (e.g. holding recess 83) configured to receive engaging members 32 of cap 30. In an example, extending portion 25 may include four connecting members 80 and cap 30 may have four engaging members 32 engageable therewith. In another example, extending portion 25 may include four connecting members 80 circumferentially equally spaced (e.g., 90 degrees apart) around the extending portion and cap 30 may have four engaging members 32 circumferentially equally spaced (e.g., 90 degrees apart) around the inner circumference of the cap. In other examples, there could be 2, 3, 4, 5, 6, 7, or 8 connecting members and corresponding engaging members on such an extending portion and cap.

In an example, connecting members (e.g., connecting members 80) of an extending portion (e.g., extending portion 25) and engaging members (e.g., engaging members 32) of a cap (e.g., cap 30) engageable with each other may be disposed evenly circumferentially around their respective surfaces. In another example, such connecting members and engaging members may be disposed unevenly around their respective surfaces. Also, the shape, size, or depth of a plurality of connecting members may be different from each other. Similarly, the shape, size, or depth of a plurality of engaging members may be different from each other. Further, some of the particular connecting members and engaging members diametrically opposed to each other and directly engaging each other may have distinct shapes relative to other such pairs circumferentially separated from each other.

Also, the housing described above (e.g., housing 20) may be formed of polypropylene (PP) plastic, for example. Seal 200 described above may be formed of low density polyethylene (LDPE). The housing (e.g., housing 20) and an outer portion of the cap (e.g., cap 30) described above may be formed of acrylonitrile butadiene styrene (ABS) plastic, for example. An inner portion of the cap (e.g., cap 30) described above may be formed of PP plastic, for example.

As described above, dispenser 10 may include a seal having a resilient force in a direction opposite knob 100 such that an axial force of 5 kgf may overcome such resilient force to allow engaging members (engaging members 32) of a cap (e.g., cap 30) to move axially past any retaining members (e.g., connecting members 80) to allow rotation of the cap and a release through recesses (e.g., recess 81, recess 91) of a holder (e.g., housing 20). Dispenser 10 may have dimensions of 133 mm x 21 mm x 21 mm, for example.

### Second Exemplary Embodiment of Dispenser

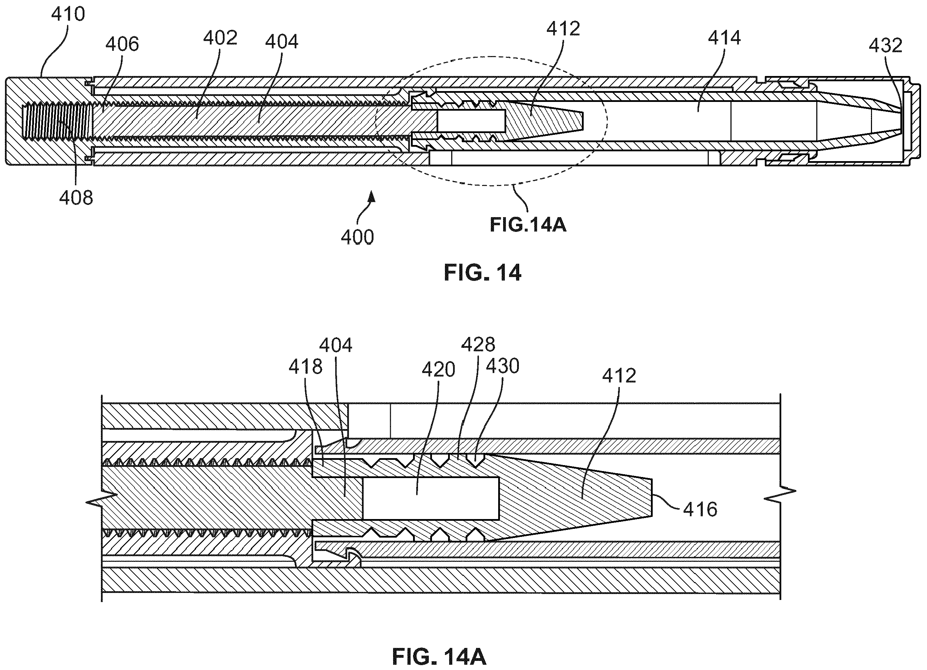

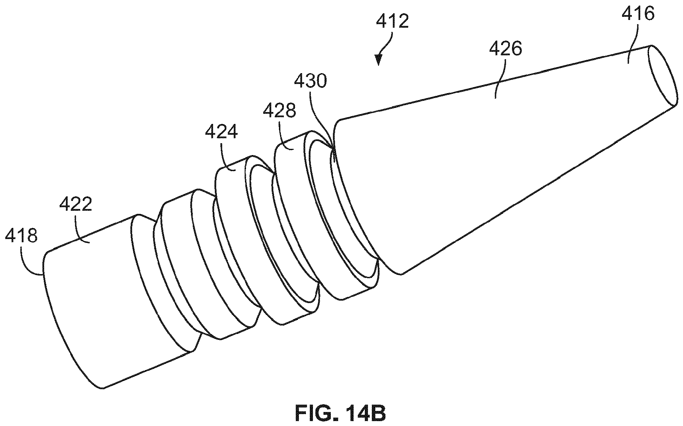

Referring now to FIG. 14, an alternative embodiment of dispenser in accordance with the invention described herein is identified generally as 400. Dispenser 400 has a similar structure as dispenser 10 and operates in a similar manner as dispenser 10 described above. Accordingly, only the differences between dispenser 10 and dispenser 400 are described in detail herein. The plunger 402 of dispenser 400 includes a shaft 404 with threads 408 that engage an interior threaded surface 406 of an activation mechanism 410. The plunger 402 further includes a plunger tip 412 that is frictionally retained on an end of shaft 404 opposite threads 408. The plunger tip 412 is positioned within a reservoir 414 of the dispenser 400.

As shown in FIGS. 14A and 14B, the plunger tip 412 has a first end 416 and a second end 418. A cavity 420 (FIG. 14A) extends from the second end 418 through the axial center of the plunger tip 412 approximately two-thirds of the way toward the first end 416. The end of shaft 404 is positioned in a portion of cavity 420 near the second end 418 to join the plunger tip 412 to the shaft 404. As best shown in FIG. 14B, plunger tip 412 has a cylindrical section 422 positioned near second end 418, a ribbed section 424 adjacent cylindrical section 422, and a frustoconical section 426 extending from ribbed section 424 to the first end 416. As shown in FIG. 14A, shaft 404 extends into cavity 420 in the cylindrical section 422 and does not appreciably extend into the portion of cavity 420 within the ribbed section 424. The ribbed section 424 includes a plurality of alternating ribs, one of which is identified as 428, and grooves, one of which is identified as 430. The alternating ribs 428 and grooves 430 and the material from which the plunger tip 412 is constructed allow the plunger tip 412 to compress when a force is applied to the first end 416 in a direction toward the second end 418 and expand back to its original configuration when the force is no longer applied to the first end 416. The plunger tip 412 is preferably constructed from a resilient material, for example a food-safe elastomer such as silicone rubber or LDPE.

In use, activation mechanism 410 is rotated in a similar manner as described above with respect to dispenser 10 to move plunger 402 through reservoir 414 toward a dispensing orifice 432. As plunger tip 412 advances through reservoir 414 it exerts a force on the fluid within reservoir 414 in a direction toward dispensing orifice 432. As known, the fluid exerts a reaction force on plunger tip 412 in a direction from first end 416 to second end 418. If the reaction force exerted on plunger tip 412 reaches a certain level, the reaction force causes the ribbed section 424 of plunger tip 412 to compress. The open cavity 420, resilient material from which the plunger tip 412 is constructed, and the ribs 428 and grooves 430 allow the plunger tip 412 to compress. As it compresses, the plunger tip 412 stores energy. This energy may be released as the plunger tip 412 expands back to its original structure prior to being compressed. As the energy is released, the plunger tip 412 displaces a portion of the fluid within reservoir 414 to push the fluid out of the reservoir 414 and through the dispensing orifice 432. The plunger tip 412 may be more likely to compress when the fluid or concentrate within reservoir 414 has a high viscosity. By compressing, the plunger tip 412 makes it easier for a user to rotate the activation mechanism 410 when dispensing a viscous fluid. For example, less torque may be required for the user to rotate the activation mechanism 410 to move the shaft 404 of the plunger 402 a desired distance.

### Third Exemplary Embodiment of Dispenser

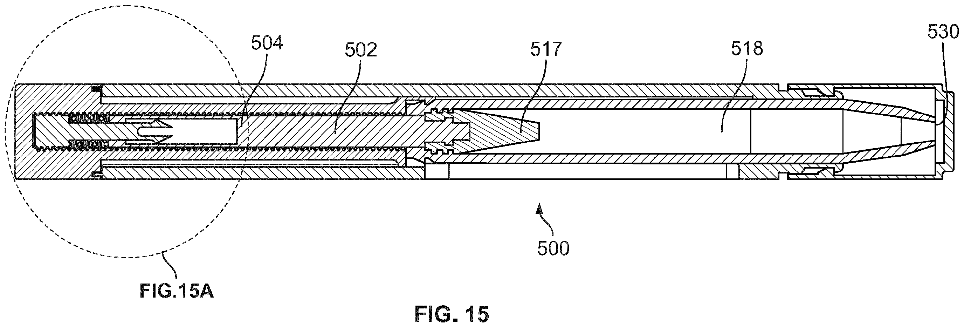

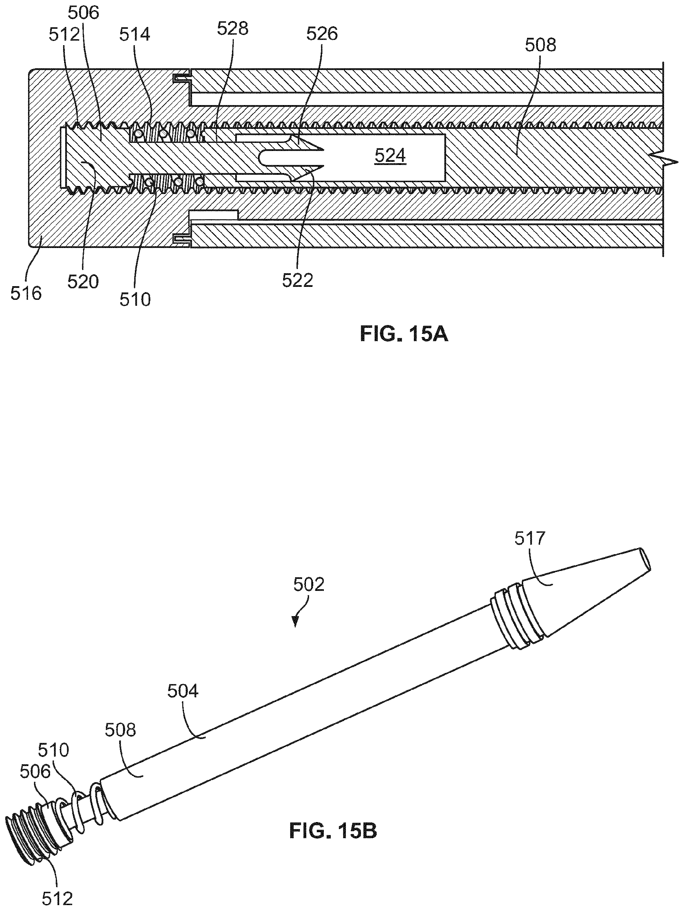

Referring now to FIG. 15, an alternative embodiment of dispenser in accordance with the invention described herein is identified generally as 500. Dispenser 500 has a similar structure as dispenser 10 and operates in a similar manner as dispenser 10 described above. Accordingly, only the differences between dispenser 10 and dispenser 500 are described in detail herein. The plunger 502 of dispenser 500 includes a shaft 504 with a first section 506 and a second section 508, shown in FIGS. 15A and 15B. A spring 510 is positioned between the first section 506 and the second section 508. The spring 510 is a compression spring that acts to bias the second section 508 away from the first section 506. The first section 506 includes threads 512 that engage an interior threaded surface 514 of an activation mechanism 516. A plunger tip 517 is frictionally retained on an end of the second section 508 of shaft 504 opposite threads 512. The plunger tip 517 is positioned within a reservoir 518 of the dispenser 500.

As shown in FIG. 15A, the first section 506 of shaft 504 has a first end 520 that includes the threads 512 and a second end 522 that is positioned within a cavity 524 of the second section 508. The second end 522 includes barbs 526 that are operable to flex radially inward to allow the second end 522 to be inserted through an opening 528 at an end of second section 508. Once inside the cavity 524, the barbs 526 expand to retain them within the cavity 524 coupling first section 506 to second section 508. The cavity 524 is sized to allow the second section 508 to move toward and away from the threads 512 of the first section 506. For example, the second section 508 may move toward the threads 512 compressing the spring 510. The second section 508 may move away from the threads 512 until the barbs 526 contact the surface surrounding opening 528, which prevents further movement of the second section 508 in a direction away from the threads 512. The spring 510 exerts a force on the second section 508 biasing the second section 508 away from the threads 512.

In use, activation mechanism 516 is rotated in a similar manner as described above with respect to dispenser 10 to move plunger 502 through reservoir 518 toward a dispensing orifice 530. As plunger tip 517 advances through reservoir 518 it exerts a force on the fluid within reservoir 518 in a direction toward dispensing orifice 530. As known, the fluid exerts a reaction force on plunger tip 517 in a direction from plunger tip 517 to first section 506. If the reaction force exerted on plunger tip 517 reaches a certain level, the reaction force causes the second section 508 to move toward the first section 506 thereby compressing the spring 510. As the spring 510 compresses, it stores energy. This energy may be released as the spring 510 expands back to its original state prior to being compressed. As the energy is released, the spring 510 moves the second section 508 away from the first section 506 thereby advancing the plunger tip 517 through the reservoir 518 toward the dispensing orifice 530. As the plunger tip 517 moves, it displaces a portion of the fluid within reservoir 518 to push the fluid out of the reservoir 518 and through the dispensing orifice 530. The spring 510 may be more likely to compress when the fluid or concentrate within reservoir 518 has a high viscosity. By compressing, the spring 510 makes it easier for a user to rotate the activation mechanism 516 when dispensing a viscous fluid. For example, less torque may be required for the user to rotate the activation mechanism 516 to move the first section 506 of the shaft 504 a desired distance.

### Fourth Exemplary Embodiment of Dispenser

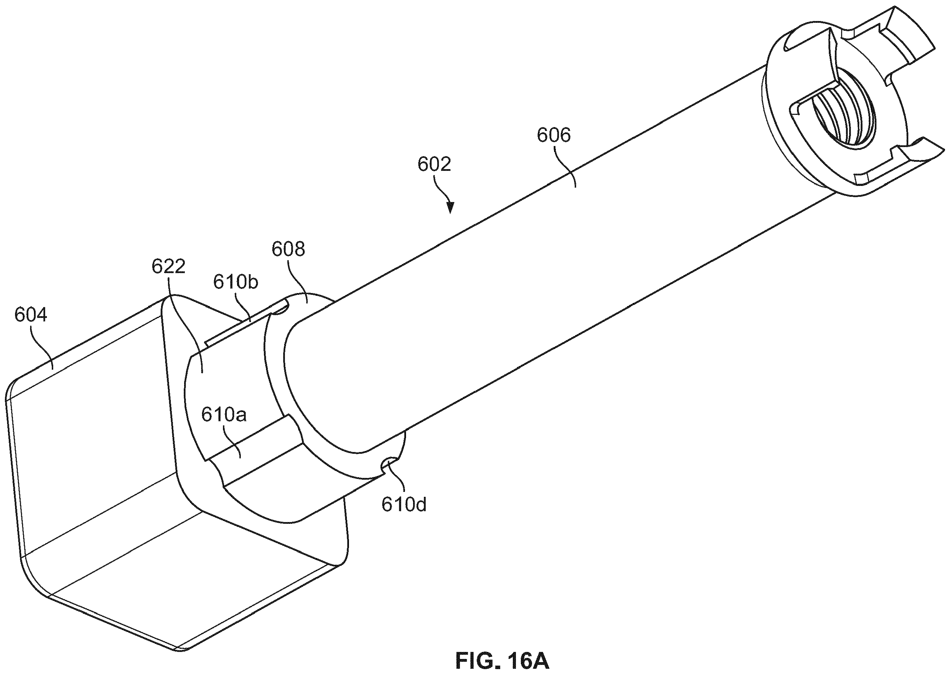

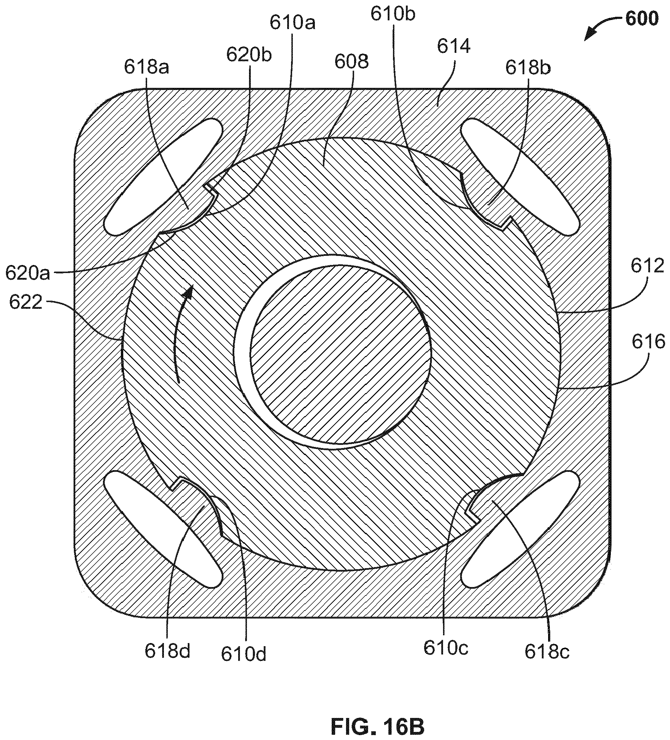

FIGS. 16A and 16B show components of a fourth embodiment of dispenser 600 in accordance with the invention described herein. Dispenser 600 may be substantially similar to any of dispensers 10, 400, or 500 other than the differences described herein. Dispenser 600 has an activation mechanism 602, shown in FIG. 16A, with a knob 604 and a sleeve 606. The knob 604 is substantially similar to the knob 100 described above. The sleeve 606 is substantially similar to the sleeve 300 described above except that sleeve 606 includes a ring 608 at its base adjacent knob 604. Four recesses 610a-d (see FIG. 16B) are formed in an outer surface of the ring 608. As shown in FIG. 16B, the ring 608 is received within a cylindrical recess 612 of a housing 614 of dispenser 600.

The housing 614 includes an inner wall 616 that defines cylindrical recess 612. Four protrusions or teeth 618a-d extend radially inward from the inner wall 616. The teeth 618a-d are spaced equidistant from each other around the circumference of the inner wall 616. The teeth 618a-d have a substantially similar structure. Accordingly, only tooth 618a is described in detail herein. Tooth 618a has a curved surface 620a that extends outward from the inner wall 616, and a flat surface 620b that extends between an end of the curved surface 620a back to the inner wall 616. Each of the recesses 610a-d of activation mechanism 602 is formed with a shape that matches the shape of the teeth 618a-d such that the teeth 618a-d are configured to be received within the recesses 610a-d as shown in FIG. 16B.

The recesses 610a-d and teeth 618a-d are configured to provide tactile or audible feedback to the user when activation mechanism 602 is rotated in a similar manner as described above with respect to rotation of activation mechanism 79. For example, when activation mechanism 602 is rotated in the clockwise direction when viewed as shown in FIG. 16B, the curved surface 620a of each of the teeth 618a-d will gradually engage an outer surface 622 of activation mechanism 602 allowing the outer surface 622 to move past the teeth 618a-d. When the activation mechanism 602 has rotated approximately 90 degrees from the position shown in FIG. 16B, the recess 610a will receive the tooth 618b, the recess 610b will receive the tooth 618c, the recess 610c will receive the tooth 618d, and the recess 610d will receive the tooth 618a. As this happens, the teeth 618a-d may snap into the recesses 610a-d providing audible feedback to the user that 90 degree rotation of the activation mechanism 602 has occurred. Further, the user may feel the teeth 618a-d moving into the recesses 610a-d in the form of a vibration or an altered amount of torque necessary to rotate the activation mechanism 602 providing tactile feedback to the user. Similar to as described above with respect to activation mechanism 79, this audible and/or tactile feedback alerts the user to the fact that the activation mechanism 602 has rotated approximately 90 degrees, which correlates with an amount of concentrate that has been dispensed from the dispenser 600.

The recesses 610a-d and teeth 618a-d also substantially prevent rotation of activation mechanism 602 in the counter-clockwise direction when viewed as shown in FIG. 16B. As shown in FIG. 16B, the flat surface 620b of the tooth 618a engages the mating flat surface bounding the recess 610a to substantially prevent rotation of the activation mechanism 602 in the counter-clockwise direction. The flat surface 620b serves as a stop to prevent rotation in the counter-clockwise direction. Flat surfaces of the teeth 618b-d further engage mating flat surfaces bounding the recesses 610b-d to prevent substantial rotation of the activation mechanism 602 in the counter-clockwise direction. Preventing substantial rotation in the counter-clockwise direction ensures that the user can only rotate the activation mechanism 602 an appreciable distance in the direction that causes the plunger to advance toward the dispensing orifice (i.e., the clockwise direction when viewed as shown in FIG. 16B) and dispense concentrate from the dispenser 600. This ensures that the user does not inadvertently move the plunger a great distance backward away from the dispensing orifice. Thus, the dispenser 600 is designed so that the activation mechanism 602 can only be rotated an appreciable distance in one direction with rotation in the opposite direction substantially blocked. The dispenser 600 may allow some rotation in the counter-clockwise direction when the teeth 618a-d are positioned between adjacent recesses 610a-d. For example, if the activation mechanism 602 is rotated clockwise less than 90 degrees from the position shown in FIG. 16B (i.e., rotated less than the distance required for recess 610a to fully receive tooth 618b), the activation mechanism 602 may be rotated counter-clockwise back to the position shown in FIG. 16B, but may not be rotated further counter-clockwise from the position shown in FIG. 16B. Alternative embodiments are within the scope of this invention that would prevent any counter-clockwise rotation of the activation mechanism 602 (e.g., a ratcheting system) such that the activation mechanism 602 can only move in one direction, the direction that causes the plunger to advance toward the dispensing orifice. Dispensers 400 and 500 may include an activation mechanism similar to either activation mechanism 79 or activation mechanism 602.

For the purposes of promoting an understanding of the principles of the invention, reference is made above to embodiments of the invention and specific language describing the same. It will nevertheless be understood that no limitation of the scope of the invention is thereby intended, and any alterations and further modifications in the described embodiments, and any further applications of the principles of the invention as illustrated therein as would normally occur to one skilled in the art to which the invention relates are contemplated and protected.

From the foregoing it will be seen that this invention is one well adapted to attain all ends and objectives herein-above set forth, together with the other advantages which are obvious and which are inherent to the invention.

Since many possible embodiments may be made of the invention without departing from the scope thereof, it is to be understood that all matters herein set forth or shown in the accompanying drawings are to be interpreted as illustrative, and not in a limiting sense.

While specific embodiments have been shown and discussed, various modifications may of course be made, and the invention is not limited to the specific forms or arrangement of parts and steps described herein, except insofar as such limitations are included in the following claims. Further, it will be understood that certain features and subcombinations are of utility and may be employed without reference to other features and subcombinations. This is contemplated by and is within the scope of the claims.

## Claims

1. A dispenser comprising:
a housing defining a reservoir configured to retain a liquid, the housing further defining a dispensing orifice in fluid communication with the reservoir;
a plunger configured to move through the reservoir toward the dispensing orifice to dispense the liquid through the dispensing orifice; and
an activation mechanism coupled to the plunger, the activation mechanism configured to be moved by a user, wherein movement of the activation mechanism in a first direction causes the plunger to move toward the dispensing orifice, and wherein at least one of the activation mechanism and the housing is configured to provide feedback to a user when the activation mechanism moves a predetermined distance in the first direction.

2. The dispenser of claim 1, wherein the plunger comprises a shaft with threads that engage a threaded surface of the activation mechanism, and wherein rotation of the activation mechanism in the first direction causes the plunger to move toward the dispensing orifice.

3. The dispenser of claim 2, wherein the activation mechanism comprises a knob and a sleeve, wherein the threaded surface of the activation mechanism is an interior surface of the sleeve.

4. The dispenser of claim 2 or 3, wherein the shaft of the plunger has a first section and a second section, the first section comprising the threads that engage the threaded surface of the activation mechanism, wherein a spring is positioned between the first section and the second section, the spring being operable to compress as the plunger moves toward the dispensing orifice.

5. The dispenser of any of claims 1-3, wherein the plunger comprises a shaft and a plunger tip coupled to the shaft, wherein the plunger tip is positioned in the reservoir, wherein the plunger tip is resilient, and wherein the plunger tip is configured to compress as the plunger moves toward the dispensing orifice.

6. The dispenser of any of claims 1-5, wherein one of the activation mechanism and the housing comprises a tooth that engages a surface of the other of the activation mechanism and the housing to provide the feedback to the user.

7. The dispenser of any of claims 1-6, wherein the feedback is a tactile feedback or an audible feedback.

8. The dispenser of any of claims 1-7, wherein the housing comprises a stop that is configured to engage the activation mechanism to prevent movement of the activation mechanism in a second direction that is opposite to the first direction.

9. The dispenser of any of claims 1-8, further comprising a cap that is releasably coupled to the housing for selectively covering and uncovering the dispensing orifice, and further comprising a resilient seal coupled to the cap and configured to seal between the housing and the cap to inhibit fluid communication between the dispensing orifice and an ambient environment when the cap is coupled to the housing.

10. The dispenser of claim 9, wherein the seal is configured to provide a biasing force between the cap and the housing in a direction toward the cap from the housing when the cap is coupled to the housing.

11. The dispenser of claim 9 or 10, wherein the housing comprises a plurality of connecting members configured to engage a plurality of engaging members of the cap to connect the housing to the cap and to seal the housing.

12. The dispenser of claim 11, wherein a first connecting member of the plurality of connecting members defines a holding recess configured to receive an engaging member of the plurality of engaging members.

13. The dispenser of claim 12, wherein the first connecting member comprises a circumferentially extending portion, a tooth and an axially extending portion defining the holding recess, wherein an entry recess is defined by the tooth, an axially extending portion of a second connecting member and a bottom circumferentially extending recess bounding portion of the first connecting member.

14. A method for dispensing a liquid from a dispenser comprising a housing defining a reservoir containing the liquid, the housing further defining a dispensing orifice in fluid communication with the reservoir, a plunger configured to move through the reservoir, and an activation mechanism coupled to the plunger, the method comprising:
moving the activation mechanism in a first direction to cause the plunger to move through the reservoir toward the dispensing orifice and dispense a portion of the liquid through the dispensing orifice; and
receiving feedback from at least one of the activation mechanism and the housing when the activation mechanism has moved a predetermined distance in the first direction.

15. The method of claim 14, wherein one of the activation mechanism and the housing comprises a tooth that engages a surface of the other of the activation mechanism and the housing to provide the feedback to the user, and wherein the feedback is a tactile feedback or an audible feedback.
